**Europäisches Patentamt**

**European Patent Office**

**Office européen des brevets**

(19)

(11) Veröffentlichungsnummer: **0 009 129**
A1

# (12) EUROPÄISCHE PATENTANMELDUNG

(21) Anmeldenummer: **79103089.3**

(22) Anmeldetag: **22.08.79**

(51) Int. Cl.³: **G 05 D 23/24,** A 61 B 5/00

(30) Priorität: 23.08.78 DE 2836883
16.08.79 DE 2933236

(43) Veröffentlichungstag der Anmeldung: **02.04.80**
**Patentblatt 80/7**

(71) Anmelder: **SIEMENS AKTIENGESELLSCHAFT Berlin und München, Postfach 22 02 61, D-8000 München 22 (DE)**

(72) Erfinder: **Biller, Max, Hindenburgstrasse 49, D-8520 Erlangen (DE)**
Erfinder: **Reichenberger, Helmut, Dr. Dipl.-Phys., Irisstrasse 8, D-8501 Brand/Eckental (DE)**
Erfinder: **Zachmann, Wilfried, Nöttinger Strasse 41, D-7537 Remchingen (DE)**

(84) Benannte Vertragsstaaten: **AT BE CH FR GB IT NL SE**

(54) Vorrichtung zur temperaturkontrollierten Erwärmung von Sonden.

(57) Die Erfindung bezieht sich auf eine Vorrichtung zur temperaturkontrollierten Erwärmung von Sonden, insbesondere von Meßwertaufnehmern zur transkutanen Sauerstoffbestimmung, bestehend aus Sonde, z. B. Aufnehmer mit Meßelektroden od. dgl., sowie zugehörigem Betriebsgerät. Sonden, die für die Messung auf eine gegenüber der Umgebungstemperatur höhere Temperatur aufgeheizt werden, benötigen im allgemeinen neben der Heizeinrichtung separate Temperaturfühler, mit denen eine Regelgröße für die Versorgungsschaltung im Betriebsgerät (25) erzeugt wird. Gemäß der Erfindung enthält ein Meßwertaufnehmer (1) als Sonde eine Widerstandsstrecke (23) mit definiertem, temperaturabhängigem elektrischem Widerstand (36), welche gleichzeitig die Funktion einer Heizung und eines Temperaturfühlers erfüllt, wird im Betriebsgerät (25) der Wert (R$\vartheta$) des temperaturabhängigen Widerstandes (36) der Widerstandsstrecke (23) mit dem Wert (R$_s$) eines vorgebbaren Widerstandes (37) verglichen und liefert das Betriebsgerät (25) einen veränderbaren Heizstrom, mit dem die Temperatur der Widerstandsstrecke (23) und damit dem Wert (R$\vartheta$) des temperaturabhängigen Widerstandes (36) an den vorgegebenen Sollwert (R$_s$) angepaßt wird. Damit ist eine Beheizung und Regelung im Körpertemperaturbereich mittels Gleichstrom-Meßbrücke möglich.

SIEMENS AKTIENGESELLSCHAFT          Unser Zeichen

Berlin und München                  VPA 78 P 5090 EUR Kb

## Vorrichtung zur temperaturkontrollierten Erwärmung von Sonden

Die Erfindung bezieht sich auf eine Vorrichtung zur temperaturkontrollierten Erwärmung von Sonden, insbesondere von Meßwertaufnehmern zur transkutanen Sauerstoffbestimmung, bestehend aus Sonde, z.B. einem Aufnehmer mit Elektroden, sowie zugehörigem Betriebsgerät.

Es ist bekannt, für eine transkutane Sauerstoffmessung die Haut am Meßort zu hyperämisieren. Dieses wird im allgemeinen dadurch erreicht, daß der Meßwertaufnehmer mit seinen Elektroden beheizt ist. Aus dem DE-GM 74 22 154 ist beispielsweise bereits ein solcher Meßwertaufnehmer, insbesondere für die transkutane Sauerstoffmessung, vorbekannt, bei der im Elektrodenträgerteil eine Heizwicklung mit Anschlußleitungen eingelassen ist. Dabei ist ein Ast der Anschlußleitungen zweckmäßigerweise aus einem solchen Material gebildet, das

Wht 5 Rl / 14.8.1979

zusammen mit der Heizwicklung ein Thermopaar für die Temperaturmessung bildet. Der Temperaturmeßwert wird dabei einem zugehörigen Betriebsgerät zugeführt und dient als Regelgröße zur Einstellung der Versorgungsspannung für die Heizwicklung. Die Sauerstoffelektrode nach dem genannten Gebrauchsmuster hat gegenüber anderen Meßwertaufnehmern zur transkutanen Sauerstoffmessung, bei denen eine völlig separate Heizwicklung und Temperaturmeßfühler vorhanden sind, bereits den Vorteil, daß die Zahl der notwendigen Zuleitungen für Heizung und Temperaturfühler verringert ist. Die andere Lötstelle für das Thermoelement liegt dabei im allgemeinen direkt im Betriebsgerät. Allerdings besteht immer noch der Nachteil, daß die Temperaturmeßwerterfassung nicht unmittelbar am eigentlichen Meßort erfolgen kann. Temperaturmeßort ist vielmehr die Verbindungsstelle Heizwicklung - Zuleitungsdraht; die dort gemessene Temperatur kann aber geringfügig von der mittleren Temperatur der Heizwicklung und der Meßelektrode abweichen.

Aufgabe der Erfindung ist es daher, eine Vorrichtung für die temperaturkontrollierte Erwärmung eines Meßwertaufnehmers mit Meßelektroden und zugehörigem Betriebsgerät anzugeben, bei der Temperaturerfassung und -regelung bezüglich der Einstellung von Heizstromspannung so weit wie möglich vereinfacht werden.

Die Aufgabe wird erfindungsgemäß dadurch gelöst, daß der Meßwertaufnehmer eine Widerstandsstrecke mit definiertem, temperaturabhängigem elektrischem Widerstand enthält, welche gleichzeitig die Funktion einer Heizung und eines Temperaturfühlers erfüllt, daß im Betriebsgerät der Wert des temperaturabhängigen Widerstandes der Widerstandsstrecke mit dem Wert eines

- 3 -         VPA 78 P 5090 EUR Kb

vorgebbaren Widerstandes verglichen und daß das Betriebsgerät einen veränderbaren Heizstrom liefert, mit dem die Temperatur der Widerstandsstrecke und damit der Wert des temperaturabhängigen Widerstandes an den vorgegebenen Sollwert angepaßt wird.

Dabei enthält das Betriebsgerät vorzugsweise eine Gleichstrom-Meßbrücke, deren Diagonalspannung der Differenz vom Widerstandswert der Widerstandsstrecke und Widerstandswert des Vergleichswiderstandes proportional ist und die damit unmittelbar die Temperaturabweichung des Heizelementes vom vorgegebenen Sollwert anzeigt. Die Diagonalspannung der Gleichstrommeßbrücke steuert dabei vorzugsweise über eine Verstärkerschaltung die Brückenspeisespannung im Sinne der Regelung der Versorgungsspannung für das Heizelement.

Es ist zwar vom Prinzip her bereits bekannt, aus dem Widerstandsverlauf eines Heizelementes auch eine Temperaturmeßgröße abzuleiten. Im Rahmen der Erfindung dient diese Temperaturmeßgröße aber gleichzeitig als Regelgröße, die durch Vergleich mit einem Sollwertwiderstand das Heizelement auf die gewünschte Temperatur regelt. Dieses läßt sich im gewünschten Bereich der Körpertemperatur ohne große Leistungsanforderung der zugehörigen Schaltung realisieren.

Weitere Einzelheiten und Vorteile der Erfindung ergeben sich aus der nachfolgenden Figurenbeschreibung anhand der Zeichnung in Verbindung mit weiteren Unteransprüchen.

Es zeigen:

Fig. 1 einen an einem Meßort applizierten Meßwertaufnehmer zusammen mit dem zugehörigen
Betriebsgerät in Prinzipdarstellung,

Fig. 2 den Meßwertaufnehmer mit Betriebsgerät
nach Fig. 1 schaltbildmäßig,

Fig. 3 die Versorgungsschaltung für die in Fig. 1
dargestellte Heizeinrichtung, die Teil des
Betriebsgerätes ist.

Fig. 4 die Schaltung nach Fig. 3 mit zusätzlicher
Überwachungsschaltung.

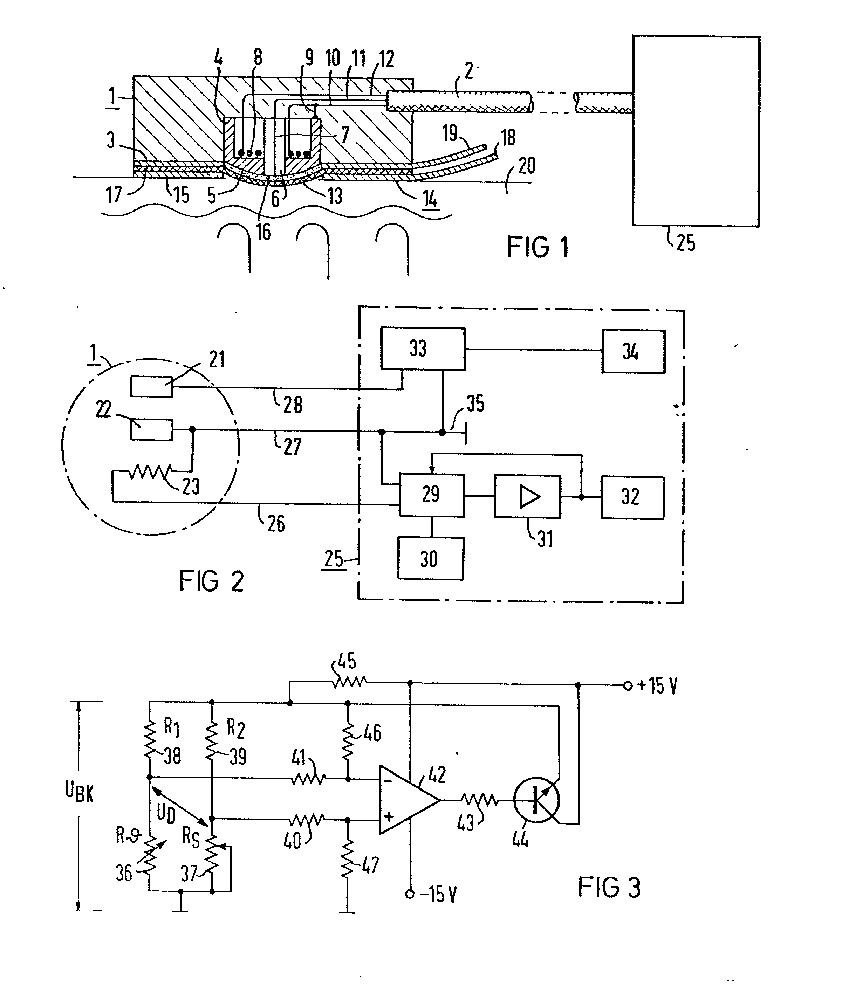

In der Fig. 1 ist mit 1 das Trägerteil eines Meßwertaufnehmers mit Meßelektroden bezeichnet, der auf der
Hautoberfläche eines Patienten als Meßort 20 für eine
transkutane Sauerstoffmessung aufgesetzt und fixiert
ist. Das Trägerteil ist dabei zweckmäßigerweise als
flache zylindrische Scheibe ausgebildet und hat einen
Anschluß 2 mit gleichzeitiger Zugentlastung für elektrische Leitungen. Der Anschluß 2 ist an ein externes
Betriebsgerät 25 angeschlossen.

Das Trägerteil 1 besteht aus gut wärmeisolierendem
Kunststoffmaterial und hat zur Applikationsseite eine
ringförmige Auflagefläche 3, die bei Applikation eine
genügende Standsicherheit gegen Verkippungen, Verschiebung od.dgl. gewährleistet. Zentrisch im Trägerteil 1
ist ein Hohlzylindertopf 4 als Anode eingegossen. Dieser Elektrodentopf 4 besteht aus Silber und ist in
geeigneter Weise mit seiner Grundfläche ausgeformt.
Die Zylinderwandung schließt mit ihrer unteren Kante
bündig mit der Ringfläche 3 des Trägerteils 1 ab. Die
Grundfläche 5 des Topfes 4 ist dabei als Teil einer Kugelfläche ausgebildet, so daß sie gegenüber der Ringfläche 3 eine konvexe Auswölbung bildet. Die konvexe Grundfläche weist etwa in der Mitte eine kleine zentrale Ausnehmung 6 auf, in der ein Draht 7 als Kathode isoliert eingesetzt ist. Der Kathodendraht 7 ist durch einen Platindraht mit 20 /um Durchmesser gebildet, dessen Stirnfläche poliert ist und als wirksame Kathodenfläche dient. Zwischen Zylinderwand des Anodentopfes 4 und dem Kathodendraht 7 ist ein Element mit einer definierten Widerstandsstrecke angeordnet, das gleichzeitig die Funktion einer Heizung und eines Temperaturfühlers erfüllt.

Als Material für die Widerstandsstrecke 8 werden beispielsweise Kupfer- oder Platindrähte verwendet. Ebenfalls möglich sind auch auf Keramiksubstraten durch Aufdampfen, elektrolytische Abscheidung od.dgl. aufgebrachte Widerstandsschichten, vorzugsweise aus Nickel. Derartige Elemente lassen sich in befriedigendem Maß gleichermaßen als Heizelement und als Temperaturmeßfühler verwenden. Dabei kommt es lediglich darauf an, daß der vom Element definierte Widerstand sowie der spezifische Temperaturkoeffizient des Widerstandsmaterials bekannt ist.

In Fig. 1 sind Anode 4, Kathode 7 und Heizwicklung über elektrische Zuleitungen 9 bis 12 mittels Kabelanschluß mit dem Betriebsgerät 25 verbunden, das sowohl den Heizspannungsversorgungsteil als auch den Meßwertverarbeitungsteil beinhaltet. Dabei sind 9 und 11 die Meßleitungen für die Elektroden; 10 und 12 sind dagegen die einzigen Leitungen für das Heiz- und Temperaturmeßelement. Die Leitung 9 ist an die Leitung 10 angeschaltet, so daß lediglich drei Leitungen aus dem Meßwertaufnehmer herausgeführt werden müssen.

0009129

- 6 -          VPA 78 P 5090 EUR  Kb

Der Meßwertaufnehmer nach Fig. 1 ist auf der Hautoberfläche 20 als Meßort appliziert. Zwischen Elektrodenmeßfläche und Hautoberfläche 20 sind dabei für die Messung ein Elektrolyt 16 sowie eine sauerstoffdurchlässige Membran 13 angeordnet. Zwischen ringförmigem Trägerteil 3 und Haut 20 befindet sich dabei zur Fixierung des gesamten Meßwertaufnehmers eine Klebehalterung 14. Durch die konvexe Ausbildung der Anodenunterfläche 5 wird beim Aufbringen der sauerstoffdurchlässigen Membran 13 und Fixieren mittels Klebehalterung 14 gleichzeitig eine gewisse Vorspannung der formelastischen Membran erreicht. Dadurch ist in befriedigender Weise gewährleistet, daß bei Applikation mittels Klebehalterung keine Luftblasen im Elektrolyten 16 zwischen Elektrodenunterfläche 5 und Membran 13 verbleiben. Gleichzeitig ist bei einem derartigen Aufbau ein guter Wärmeübergang von Heizelement über die metallische Anode 4 zur Hautoberfläche 20 sichergestellt, so daß die Haut am Meßort aktiv hyperämisiert ist.

In der Fig. 2 sind Meßwertaufnehmer und Betriebsgerät schaltbildmäßig dargestellt: Im Meßwertaufnehmer 1 sind mit 21 und 22 die Elektroden (Kathode und Anode der polarographischen Meßzelle) und mit 23 die definierte Widerstandsstrecke bezeichnet. Der eine Anschluß der Widerstandsstrecke 23 liegt dabei auf Anodenpotential, so daß sich eine weitere Zuleitung zum Aufnehmer erübrigt. Als Anschlußleitungen zum Betriebsgerät 25 werden also ingesamt nur drei Leitungen 26 bis 28 benötigt.

Im gestrichelt angedeuteten Betriebsgerät 25 bedeuten 29 eine Gleichstrom-Brückenschaltung, 30 ein Stellglied für einen Widerstand in der Brücke, 31 eine Ver- stärkerschaltung, 33 eine Meßwerterfassungsschaltung für den polarographischen Meßstrom sowie 32 und 34 Anzeigegeräte. Im einzelnen stellt dabei die Widerstandsstrecke 23 den Meßwiderstand der Meßbrücke 29 dar, was in Fig. 3 näher erläutert wird. Mittels Stellglied 30 ist dabei ein weiterer Widerstand im Sinne einer Sollwerteinstellung veränderbar. Damit lassen sich Sollwerte für die Heizung im Körpertemperaturbereich, beispielsweise in 2°C-Schritten von 36 bis 42°C vorgeben. Die Verstärkerschaltung 31 liefert die Versorgungsspannung für die Meßbrücke 29; sie arbeitet also als Regler in dem Sinne, daß der temperaturabhängige Widerstand der Widerstandsstrecke 23 an den vorgegebenen Sollwiderstand angepaßt wird. Am ersten Anzeigegerät 32 wird die Heizleistung für die Widerstandsstrecke 23 angezeigt, die ein Maß für die Wärmeabfuhr am Meßwertaufnehmer 1 ist und damit gleichzeitig eine Aussage über die Durchblutung der Haut angibt. Die Messung des Stromes in der polarographischen Meßzelle erfolgt nach dem üblichen Prinzip der Messung kleiner Ströme. Im Betriebsgerät wird ein Anschluß der Widerstandsstrecke auf floatendes Massepotential gelegt, was mit dem Bezugszeichen 35 gekennzeichnet ist.

In der Fig. 3 sind mit 36 bis 39 vier Widerstände bezeichnet, die als Gleichstrommeßbrücke geschaltet sind. Dabei sind 38 und 39 zwei Konstantwiderstände, während 36 und 37 veränderliche Widerstände sind. Der Widerstand 36 wird im einzelnen durch die definierte Widerstandsstrecke 23 nach Fig. 2 gebildet. Sein Widerstandswert ändert sich also mit der jeweils vorliegenden Temperatur des Meßwertaufnehmers 1. Der Widerstand 37 ist dagegen ein Potentiometer, dessen Widerstandswert von der Bedienungsperson im Sinne der Einstellung eines Sollwertes veränderbar ist.

Zwischen den Widerstandszweigen der Meßbrücke fällt eine Diagonalspannung $U_D$ ab. Diese Diagonalspannung $U_D$ wird über Widerstände 40 und 41 auf einen Operationsverstärker 42 gegeben, der an eine Spannungsquelle $\mp$ 15 V angeschaltet ist. Der Ausgangswert wird über einen Widerstand 43 und einen npn-Transistor 44 in Kollektorschaltung auf den Brückenspeisespannungsversorgungszweig zurückgekoppelt. Dafür sind weiter die Widerstände 46 und 47 zur Beschaltung des Operationsverstärkers 42 notwendig. Weiterhin ist noch ein Widerstand 45 dem Brückenspeisespannungsversorgungszweig parallelgeschaltet.

Mittels der in Fig. 3 beschriebenen Schaltung wird also die Brückenspeisespannung $U_{BK}$ in Abhängigkeit von der Brückendiagonalspannung $U_D$ geregelt. Für die Schaltung nach Fig. 3 gelten im einzelnen folgende Beziehungen:

(1) $\quad U_D = U_{BK} \left( \dfrac{R_\vartheta}{R_1 + R_\vartheta} - \dfrac{R_s}{R_2 + R_s} \right)$

(2) $\quad U_{BK} = - U_D \cdot V$

wobei $R_\vartheta$ Widerstand 36, $R_s$ Widerstand 37, $R_1$ und $R_2$ die Widerstände 38, 39 gemäß Fig. 3 sowie V der Verstärkungsfaktor des Operationsverstärkers 42 bedeuten. V ist im wesentlichen aus dem Verhältnis der Widerstände 46 und 41 sowie 47 und 40 bestimmt.

Gleichung (2) in Gleichung (1) ergibt:

(3) $\quad R_\vartheta = f(T) = \dfrac{\left( \dfrac{1}{V} + \dfrac{R_s}{R_2 + R_s} \right) R_1}{1 - \dfrac{1}{V} - \dfrac{R_s}{R_2 + R_s}}$

Aus der Analyse der für Brücke und Verstärkerschaltung gültigen Gleichungen (1), (2) und (3) ergibt sich die Funktion zur gleichzeitigen Temperaturmessung und -regelung: Der Widerstand des Heizelementes $R_\vartheta$ hat im interessierenden Temperaturbereich eine bekannte Temperaturfunktion. Unter der Voraussetzung, daß die Hilfswiderstände $R_1$, $R_2$ sowie der Verstärkungsfaktor V konstant sind, läßt sich also $R_\vartheta$ allein durch $R_S$ bestimmen bzw. einregeln. Je nach Wärmeabfuhr im Meßwertaufnehmer regelt dann die Brücken- und Verstärkerschaltung die Brückenspeisespannung $U_{BK}$ und damit den Strom durch den Heizwiderstand; im Ergebnis ist also die Temperatur der Widerstandsstrecke 23 nach Fig. 2 mit Widerstand 36 unabhängig von der Wärmeabfuhr im Aufnehmer 1. Die Widerstände 40 und 41 sowie 46 und 47 zur Beschaltung des Operationsverstärkers sind jeweils gleich groß, so daß sich ein symmetrischer Differenzverstärker ergibt. Der Widerstand 43 ist im wesentlichen notwendig als Vorschaltwiderstand für den Leistungstransistor 44, wogegen der Widerstand 45 zum Anlaufen der Schaltung benötigt wird.

Beim Regelvorgang ergibt sich im wesentlichen folgender Funktionsablauf: Am Widerstand 37 wird ein Sollwert eingestellt. Über den Widerstand 45 wird von der Gleichspannungsquelle die Brücke mit einer geringen Spannung beaufschlagt. Dabei ergibt sich an der Brücke bereits eine kleine dementsprechende Diagonalspannung $U_D$, die gemäß Gleichung (2) im Verstärker 42 verstärkt wird. Der nachgeschaltete Transistor 44 wird dann durchgesteuert, so daß an der Brücke als Speisespannung eine entsprechend dem Verstärkungsgrad des Verstärkers 42 verstärkte Spannung anliegt. Es fließt also durch den Widerstand 36 ein Strom, wobei die Widerstandsstrecke entsprechend der eingespeisten Leistung aufgeheizt und dementsprechend der Wert des Widerstandes gemäß seinem Temperaturkoeffizienten geändert wird. Die Brückenspeisespannung $U_{BK}$ ist also ein Maß für die Heizleistung im Widerstand 36 und damit gleichzeitig für die im Heizwiderstand 23 nach Fig. 2 abgeführte Wärmemenge.

Diese Brückenspannung wird desto kleiner, je geringer die Differenz zwischen den Widerständen 36 und 37 und je kleiner damit die Diagonalspannung $U_D$ ist. Für den Fall, daß der Widerstandswert des Widerstandes 36 größer als der Widerstandswert des Widerstandes 37 ist, wird sich am Ausgang des Verstärkers 42 eine negative Ausgangsspannung ergeben, die den Transistor 44 sperrt. In diesem Fall fließt nur noch der Strom über den Widerstand 45, der zum Anlaufen der Brücke notwendig ist. Dieser Strom ist aber vernachlässigbar gering, so daß er nicht wesentlich zur Heizung beiträgt.

Es hat sich als zweckmäßig erwiesen, bei der Gleichstrom-Meßbrücke den Sollwiderstand $R_S$ und zugehörigen Festwiderstand $R_2$ einerseits sowie den Meß/Regelwiderstand $R_v$ und zugehörigen Festwiderstand $R_1$ jeweils gleich groß zu dimensionieren. Dadurch ergibt sich die optimale Meßempfindlichkeit der Brücke. Dabei ist vorzugsweise der Sollwiderstand etwa 10 mal so groß wie der Widerstand der Widerstandsstrecke. Bei einer derartigen Dimensionierung wird bei optimaler Empfindlichkeit ein maximal möglicher Teil der in die Brücke eingespeisten elektrischen Leistung, nämlich fast 50 %, als Heizleistung umgesetzt.

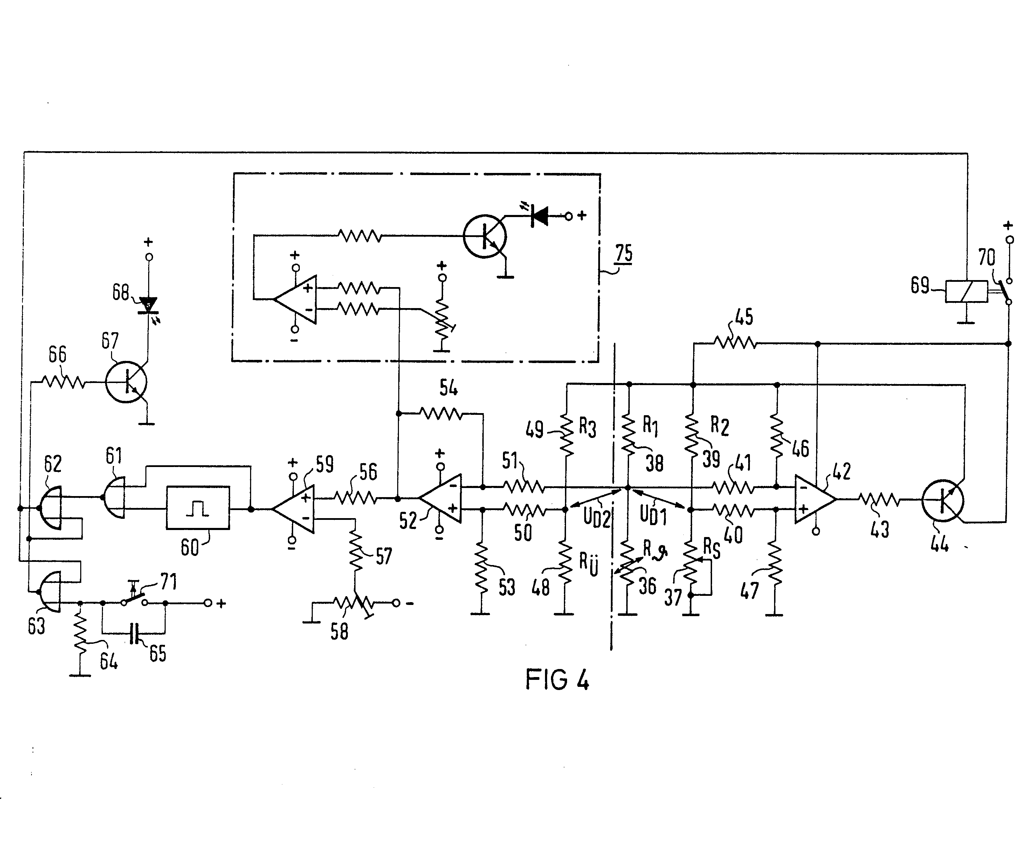

In der Fig. 4 sind mit 36 bis 47 die Elemente der Meßbrücke mit der Brückenspeisespannungs-Versorgungsschaltung nach Fig. 3 bezeichnet. Sie besteht im einzelnen wiederum aus den Widerständen 36 bis 39, die die Brücke mit dem temperaturabhängigen Widerstand $R_\vartheta$ und Soll-widerstand $R_s$ sowie den Hilfswiderständen $R_1$, $R_2$ bilden, und   dem Leistungsverstärker 42 bis 44 mit zugehörigen Beschaltungswiderständen 40 bis 43, 46 bis 47. Die Diagonalspannung dieser Meßbrücke ist in Fig. 4 mit $U_{D_1}$ bezeichnet. An den Brückenzweig mit Widerständen 36 und 38 ist ein zusätzlicher Brückenzweig mit Widerständen 48 und 49 angeschaltet. Die zwischen den Widerstandszweigen der durch die Widerstände 36, 38, 48, 49 gebildeten Meßbrücke abfallende Diagonalspan-nung $U_{D_2}$ wird über die Widerstände 50, 51 auf die Eingänge eines Operationsverstärkers 52 gegeben, der als Differenzverstärker mit Widerständen 53 und 54 beschaltet ist. Das so im Differenzverstärker 52 verstärkte Brückensignal wird über einen Widerstand 56 auf einen als Komparator geschalteten Operationsverstärker 59 gegeben. Am Vergleichseingang des Komparators 59 liegt über den Widerstand 57 sowie ein Potentiometer 58 eine einstellbare Vergleichsspannung als Referenzwert. Dem Komparator 59 ist ein monostabiler Multivibrator 60 mit einstellbarer Impulszeit nachgeschaltet, von dem ein logisches Verknüpfungsglied 61 gleichzeitig mit dem Komparatorsignal angesteuert wird. Dieses Verknüpfungsglied 61 ist ein NOR-Gatter. Vom NOR-Gatter 61 wird der erste Eingang eines logischen Verknüpfungsgliedes 62 angesteuert, dessen zweiter Eingang mit dem Ausgang eines zweiten Verknüpfungsgliedes 63 verbunden ist. Der erste Eingang des Verknüpfungsgliedes 63 ist wiederum an das Ausgangssignal des Verknüpfungsgliedes 62, während der zweite Eingang über einen Schalter 71 mit angeschalteter RC-Kombination 64, 65 an Betriebsspannung ange-

- 12 -    VPA 78 P 5090 EUR  Kb

schlossen ist. Die logischen Verknüpfungsglieder 62/ 63 bilden zusammen ein sog. RS-Flipflop. Vom Ausgang des ersten Verknüpfungsgliedes 62 wird ein Relais 69 gesteuert, mit dem der Leistungsverstärker 42 bis 44 des Brückenspeisekreises über einen Schalter 70 an die Betriebsspannung angeschaltet ist. Vom Ausgang des zweiten Verknüpfungsgliedes 64 wird über einen Widerstand 66 ein Schalttransistor 67 angesteuert, in dessen Kollektorstrecke eine Lumineszenzdiode 68 liegt.

Die so beschriebene, komplettierte Schaltung hat folgende Funktion:

Der Leistungsverstärker 42 bis 44 wird von der Diagonalspannung $U_{D_1}$ der Gleichstrom-Meßbrücke 36 bis 39 gesteuert und speist eine entsprechende Brückenspeisespannung $U_{BK}$ in die Meßbrücke ein. Wie beschrieben, ist durch die Schaltglieder 40 bie 47 ein Proportional-Regler realisiert, wobei aber aufgrund der Regelabweichung des Reglers kein vollständiger Brückenabgleich erreicht werden kann. Bei Normalbetrieb ist daher die in der zusätzlich angeschalteten Meßbrücke aus den Widerständen 36, 38, 48, 49 auftretende Diagonalspannung $U_{D_2}$ gegen Massepotential positiv. Dabei sind die Widerstände 48 und 49 zweckmäßigerweise so gewählt, daß $R_3$ dem Widerstand $R_2$ entspricht und $R_U$ gleich oder größer dem Maximalwert von $R_S$ entsprechend gewählt wird.

Bei Ausfall der Regelschaltung kann die Heizstrecke des Abnehmers entweder zu kalt oder aber zu heiß sein. Da mit der Überwachungsschaltung primär eine Schädigung der Haut durch Übererwärmung verhindert werden soll, wird zunächst lediglich der Fall einer Übertemperatur beachtet: Bei ständigem Stromfluß durch die Heizstrecke 36 mit Widerstand $R_U$ steigt aufgrund des positiven Temperaturkoeffizienten der Widerstandswert über den Widerstandswert $R_{\ddot{u}}$ des Widerstandes 48. Die Diagonalspannung $U_{D_2}$ sowie die Ausgangsspannung am Differenzverstärker 52 werden dann negativ. Dieser Wert wird im Komparator 59 mit einer mittels Potentiometer 58 einstellbaren Referenzspannung verglichen. Bei Unterschreiten der durch die Referenzspannung festgelegten Schwelle kippt der Komparator 59 in seine negative Lage. Durch dessen Ausgangssignal wird der einstellbare Monoflop 60 gesetzt. Die Impulsdauer des Monoflops 60 ist bei beispielsweise 5 Sekunden eingestellt. Ist nach Ablauf dieser Verzögerungszeit die Störung in der Regelschaltung nicht beseitigt, wird über das Gatter 61 das RS-Flipflop 62/63 vom ersten in den zweiten Zustand geschaltet. Das Relais 69 fällt ab und der Schalter 70 wird geöffnet; die Betriebsspannung ist unterbrochen. Über den Schalttransistor 67 wird die Leuchtdiode 68 zur Anzeige der Störung aktiviert.

Im zweiten Brückenkreis mit den Widerständen 36, 38, 48, 49 wurde zweckmäßigerweise $R_3 = R_2$ und $R_{\ddot{u}} = R_s$ gewählt. Eine andere Wahl von $R_3$ und $R_{\ddot{u}}$ ist prinzipiell möglich; in einem solchen Fall muß die Schwellenspannung am Potentiometer 58 für das Umschalten des Komparators 59 auf einen entsprechenden Wert eingestellt werden.

Während im beschriebenen Ausführungsbeispiel im wesentlichen der Fall der Übertemperatur betrachtet wurde, kann durch Zuschalten eines weiteren Komparators und Vergleich des Signals von Verstärker 52 mit einem positiven Referenzwert auch eine Untertemperatur des Widerstandes $R_{\downarrow}$ erfaßt und signalisiert werden. Eine solche Schaltungsgruppe 75 ist fakultativ im gestrichelten Kasten der Figur angedeutet.

Bei erstmaliger oder erneuter Inbetriebnahme der Heizvorrichtung wird durch die Kondensatorkopplung 65 gewährleistet, daß das RS-Flipflop 62/63 in dem Zustand gesetzt ist, der ein Einschalten des Relais 69 bewirkt. Nach Beseitigung der Störung kann die Regelung durch Schließen des Schalters 71 über einen Drucktaster od.dgl. wieder in Betrieb gesetzt werden.

Mit der Schaltung nach Fig. 4 ist also in vorteilhafter Weiterbildung der Erfindung eine wirksame Temperaturüberwachungsvorrichtung geschaffen worden, ohne daß etwa ein weiterer Temperaturmeßfühler benötigt wird.

Anhand der Zeichnung wurde speziell die temperaturkontrollierte Beheizung einer transkutanen Sauerstoffelektrode beschrieben. In anderen Ausführungsformen sind mit einer derartigen erfindungsgemäßen Vorrichtung auch andere Sonden, beispielsweise Handstücke für die Dentaltechnik, auf geregelte Konstanttemperatur unabhängig von der jeweils vorliegenden Wärmeabfuhr beheizbar.

Patentansprüche

1. Vorrichtung zur temperaturkontrollierten Erwärmung von Sonden, insbesondere von Meßwertaufnehmern zur transkutanen Sauerstoffbestimmung, bestehend aus Sonde, z.B. einem Aufnehmer mit Elektroden, sowie zugehörigem Betriebsgerät, d a d u r c h   g e k e n n z e i c h - n e t , daß die Sonde (1) eine Widerstandsstrecke(23) mit definiertem, temperaturabhängigem elektrischem Widerstand (36) enthält, welche gleichzeitig die Funktion einer Heizung und eines Temperaturfühlers erfüllt, daß im Betriebsgerät (25) der Wert ($R_\vartheta$) des temperaturabhängigen Widerstandes (36) der Widerstandsstrecke (23) mit dem Wert ($R_s$) eines vorgebbaren Widerstandes (37) verglichen wird und daß das Betriebsgerät (25) einen veränderbaren Heizstrom liefert, mit dem die Temperatur der Widerstandsstrecke (23) und damit der Wert ($R_\vartheta$) des temperaturabhängigen Widerstandes (36) an den vorgegebenen Sollwert ($R_s$) angepaßt wird.

2. Vorrichtung nach Anspruch 1, d a d u r c h   g e k e n n z e i c h n e t ,   daß das Betriebsgerät (25) eine Gleichstrom-Meßbrücke (29, 36 bis 39) enthält, deren Diagonalspannung ($U_D$) der Differenz vom Wert ($R_\vartheta$) des Widerstandes (36) der Widerstandsstrecke (23) und Widerstandswert ($R_s$) des Vergleichswiderstandes (37) proportional ist und die damit unmittelbar die Temperaturabweichung des Heizelementes vom vorgegebenen Sollwert angibt.

3. Vorrichtung nach Anspruch 1 und 2, d a d u r c h   g e k e n n z e i c h n e t ,   daß die Diagonalspannung ($U_D$) der Gleichstrommeßbrücke (29, 36 bis 39) über eine Verstärkerschaltung (42 bis 47) die Brückenspeisespannung ($U_{BK}$) als Heizelement-Versorgungsspannung steuert.

4. Vorrichtung nach Anspruch 1 bis 3, d a d u r c h g e k e n n z e i c h n e t , daß in der Gleichstrom-Meßbrücke (36 bis 39) Sollwiderstand ($R_s$) und zugehöriger Festwiderstand ($R_2$) einerseits und Meß-/Regelwiderstand ($R_\vartheta$) und zugehöriger Festwiderstand ($R_1$) andererseits jeweils die gleiche Größenordnung haben und daß der Sollwiderstand ($R_s$) wesentlich größer als der Meß-/Regelwiderstand ($R_\vartheta$), vorzugsweise etwa 10 mal so groß, ist.

5. Vorrichtung nach Anspruch 1 bis 3, d a d u r c h g e k e n n z e i c h n e t , daß die Widerstandsstrecke (23) mit einem Anschluß auf dem Potential einer der Elektroden (21, 22) liegt.

6. Vorrichtung nach Anspruch 5, d a d u r c h g e k e n n z e i c h n e t , daß das Potential ein floatendes Massepotential ist.

7. Vorrichtung nach Anspruch 3, d a d u r c h g e k e n n z e i c h n e t , daß die Verstärkerschaltung (42 bis 44, 46, 47) als rückgekoppelter Leistungsverstärker ausgebildet ist, der aus einem Operationsverstärker (42) besteht, wobei im Rückkopplungszweig des Operationsverstärkers ein Leistungstransistor (44) in Kollektorschaltung geschaltet ist, dessen Basis-Emitter-Strecke im durchgeschalteten Zustand Teil des Rückkopplungszweiges ist.

8. Vorrichtung nach Anspruch 1, d a d u r c h g e k e n n z e i c h n e t , daß im Betriebsgerät wenigstens ein Komparator (59) zum Vergleich der Abweichung des Wertes des temperaturabhängigen Widerstandes ($R_\vartheta$) vom vorgegebenen Sollwert ($R_s$) mit vor-

gebbaren Grenzwerten vorhanden ist. wobei vom Komparator (59) bei Überschreiten der Grenzwerte ein Ausgangssignal erzeugt wird.

9. Vorrichtung nach Anspruch 2 und 8, d a d u r c h g e k e n n z e i c h n e t , daß die Gleichstrom-Meßbrücke (36 - 39) einen zusätzlichen Brückenzweig (48, 49) aufweist, wobei der temperaturabhängige Widerstand ($R_\vartheta$) der Widerstandsstrecke im Mittelzweig (36, 38) der so gebildeten Doppel-Meßbrücke (36 - 39, 48, 49) liegt und vom Mittelzweig (36, 38) und zusätzlichen Brückenzweig (48, 49) eine zweite Meßbrücke (36, 38, 48, 49) gebildet wird, deren Diagonalspannung ($U_{D2}$) das Komparatorsignal ist.

10. Vorrichtung nach Anspruch 9, d a d u r c h g e k e n n z e i c h n e t , daß im zusätzlichen Brückenzweig der Vergleichswiderstand (48) für den temperaturabhängigen Widerstand ($R_\vartheta$) der Meßstrecke (36) einen Wert ($R_{\bar{u}}$) hat, der gleich oder größer dem höchsten einstellbaren Sollwert ($R_S$) des Widerstandes (37) für die Temperatureinstellung ist.

11. Vorrichtung nach Anspruch 9, d a d u r c h g e k e n n z e i c h n e t , daß die Diagonalspannung ($U_{D2}$) des zweiten Brückenkreises (36, 38, 48, 49) über einen Differenzverstärker (52) auf Komparatoren (59) zum Vergleich mit an Potentiometern (58) einstellbaren Referenzwerten gebbar ist, wobei von den Ausgangssignalen der Komparatoren (59) Anzeigeeinrichtungen (68) aktivierbar sind.

12. Vorrichtung nach Anspruch 11, d a d u r c h g e k e n n z e i c h n e t , daß der erste Komparator (59) das Brückensignal mit einem vorwählbaren,

ersten Referenzwert zum Erfassen von Übertemperaturen des temperaturabhängigen Widerstandes ($R_\psi$) vergleicht, wobei bei Überschreiten von vorgegebenen Sollwert ($R_s$) das Ausgangssignal des Komparators (59) zusätzlich eine Schaltungseinrichtung (69, 70) zur Unterbrechung der Betriebsspannung betätigt.

13. Vorrichtung nach Anspruch 11 und 12,  d a - d u r c h   g e k e n n z e i c h n e t ,  daß dem ersten Komparator (59) ein monostabiler Multivibrator (60) mit einstellbarer Impulsdauer zur Verzögerung des Komparatorsignals nachgeschaltet ist.

14. Vorrichtung nach Anspruch 11 bis 13,  d a - d u r c h   g e k e n n z e i c h n e t ,  daß Multivibratorsignal und Komparatorsignal durch Gatter (61) logisch verknüpft werden und daß vom Gatter (61) ein RS-Flipflop (62/63) von seinem ersten Zustand in seinen zweiten Zustand umgeschaltet wird, wobei durch den zweiten Zustand des Flipflop (62/63) als Anzeigeeinrichtung eine Leuchtdiode (68) aktiviert und gleichzeitig als Schalteinrichtung (69) zur Unterbrechung der Betriebsspannung ein Relais (69) betätigt wird.

15. Vorrichtung nach Anspruch 8 und 11,  d a - d u r c h   g e k e n n z e i c h n e t ,  daß im Betriebsgerät eine Schaltungsgruppe (75) mit einem zweiten Komparator zum Vergleich des verstärkten Brückensignals mit einem zweiten vorgebbaren Referenzsignal zum Erfassen von Untertemperaturen vorhanden ist.

0009129
78 P 5090    1/2

FIG 1

FIG 2

$U_{BK}$

$R_1$ 38
$R_2$ 39
R $\vartheta$
36
$U_D$
$R_S$ 37
41
40
46
47
42
45
43
44
30
29
31
32
33
34
35

+15 V
−15 V

FIG 3

FIG 4

78 P 5090    2/2

0009129

Europäisches
Patentamt

**EUROPÄISCHER RECHERCHENBERICHT**

0009129
Nummer der Anmeldung

EP 79 103 089.3

| Kategorie | Kennzeichnung des Dokuments mit Angabe, soweit erforderlich, der maßgeblichen Teile | betrifft Anspruch |
|---|---|---|
| | DE - A - 2 145 199 (K.K. TOKAI RIKA DENKI SEISAKUSHO NISHIBIWAJIMA) * ganzes Dokument * | 1-3, 8 |
| | -- | |
| | DE - B2 - 2 305 510 (SIEMENS AG) * Patentanspruch; Spalte 3, Zeilen 43 bis 48 * | 1,4 |
| | -- | |
| | US - A - 4 086 466 (R.S. SCHARLACK) * Patentansprüche; Spalte 1, Zeilen 37 bis 39 * | 1 |
| | -- | |
| P | DE - A1 - 2 724 558 (Dr. K. THOMAE GMBH) * Patentanspruch, Spalte 2, Zeile 46 bis Spalte 3, Zeile 12 * | 1-3 |
| | -- | |
| A | DE - A1 - 2 643 658 (DEUTSCHE RANCO GMBH) * Seiten 1 bis 6 * | 1,11 |
| | -- | |
| A | DE - A - 2 153 408 (BROWN, BOVERI & CIE AG) * ganzes Dokument * | |
| | -- | |
| D | DE - U - 7 422 154 (SIEMENS AG) * ganzes Dokument * & US - A - 3 998 212 | |
| | ----- | |

**EINSCHLÄGIGE DOKUMENTE**

**KLASSIFIKATION DER ANMELDUNG (Int.Cl.3)**

G 05 D   23/24
A 61 B   5/00

**RECHERCHIERTE SACHGEBIETE (Int.Cl.3)**

A 61 B   5/00
G 01 N   33/00
G 05 D   23/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

☒ Der vorliegende Recherchenbericht wurde für alle Patentansprüche erstellt.

| Recherchenort | Abschlußdatum der Recherche | Prüfer |
|---|---|---|
| Berlin | 21-12-1979 | BEYER |

EPA form 1503.1   06.78